# EUROPÄISCHE PATENTANMELDUNG

(11) **EP 4 722 692 A1**
(43) Veröffentlichungstag der Anmeldung: **08.04.2026**
(21) Anmeldenummer: 25198944.8
(22) Anmeldetag: 29.08.2025
(51) Int. Cl.: G01N 21/3504, G01N 33/00, G01N 21/61

(54) **VORRICHTUNG UND VERFAHREN ZUM MESSEN EINER CH4-KONZENTRATION IN EINEM WASSERDAMPF ENTHALTENDEN GASGEMISCH**

(30) Priorität: 04.10.2024 DE 102024128729
(71) Anmelder: Wiegleb, Gerhard, 58849 Herscheid (DE)
(72) Erfinder: Wiegleb, Gerhard, 58849 Herscheid (DE)
(74) Vertreter: Schatz, Markus Franz-Josef

(57) **Zusammenfassung**

Die Erfindung betrifft eine Vorrichtung (1) zum Messen einer CH₄-Konzentration in einem Wasserdampf enthaltenden Gasgemisch. Um die Genauigkeit einer NDIR-Messung einer CH₄-Konzentration in einem Wasserdampf enthaltenden Gasgemisch zu erhöhen, weist die Vorrichtung (1) wenigstens eine NDIR-Messeinrichtung (2) und wenigstens eine bezüglich einer Strömung des Gasgemischs durch die Vorrichtung (1) der NDIR-Messeinrichtung (2) vorgeschaltet angeordnete und für Wasserdampf selektiv permeabel ausgebildete Gasführungseinheit (6) zum Trocknen und/oder Anfeuchten des Gasgemischs auf, wobei die Gasführungseinheit (6) derart ausgebildet ist, dass ein Wassergehalt des aus ihr austretenden Gasgemischs entweder einem Wassergehalt einer die Gasführungseinheit (6) umgebenden, CH₄-freien Umgebungsluft entspricht oder um maximal 5% von dem Wassergehalt der die Gasführungseinheit (6) umgebenden, CH₄-freien Umgebungsluft abweicht.

## Beschreibung

### Technisches Gebiet

Die Erfindung betrifft eine Vorrichtung und ein Verfahren zum Messen einer CH₄-Konzentration in einem Wasserdampf enthaltenden Gasgemisch. Zudem betrifft die Erfindung eine Verwendung einer solchen Vorrichtung zum Messen einer CH₄-Konzentration in einem Wasserdampf enthaltenden Gasgemisch.

### Stand der Technik

In der Gassensorik ist die nichtdispersive Infrarotabsorption (NDIR) etabliert. Ein hierzu geeignetes NDIR-Gasmessgerät ist beispielsweise aus EP 0 794 423 A1 bekannt. Die von einer Infrarot-Strahlungsquelle emittierte Strahlung durchläuft hierbei eine Messküvette, in der sich ein Gasgemisch befindet, und trifft am entgegengesetzten Ende der Messküvette auf einen infrarotempfindlichen Gassensor. Die Abschwächung der elektromagnetischen Strahlung durch das in der Küvette eingeschlossene Gasgemisch ist nach dem Lambert-Beer'schen Gesetz ein Maß für die Konzentration einer zu messenden Gaskomponente.

NDIR-Gasmessgeräte, die auf eine spezifische Gaskomponente eines als Gasgemisch vorliegenden Messgases reagieren, können Querempfindlichkeiten zu anderen in dem Messgas enthaltenen Gaskomponenten (auch Störkomponenten genannt) aufweisen. Diese Querempfindlichkeiten müssen kompensiert werden, um korrekte Messergebnisse erhalten zu können. Dies kann beispielsweise entsprechend der Gebrauchsmusterschrift DE 20 2019 101 137 U1 erfolgen. Allerdings verursacht diese Vorgehensweise aufgrund der hierzu baulich erweiterten Messanordnung zusätzliche Kosten.

Des Weiteren stellt in einem Messgas enthaltener Wasserdampf bei NDIR-Messungen ein Problem dar, da auch hierdurch ungenaue Messergebnisse verursacht werden. Um den Wassergehalt in einem Messgas zu reduzieren und über konstant zu halten, kann das Messgas mit einem Messgaskühler gekühlt und in seiner Temperatur geregelt werden. Auch dies ist jedoch mit hohen Kosten für den Aufbau der Messanordnung verbunden.

Ein anderes Problem von NDIR-Gasmessgeräten besteht in der Langzeitdrift, insbesondere Nullpunktdrift), die durch eine regelmäßige Justage der NDIR-Gasmessgeräte behoben werden kann.

### Offenbarung der Erfindung

Eine Aufgabe der Erfindung ist es, die Genauigkeit einer NDIR-Messung einer CH₄-Konzentration in einem Wasserdampf enthaltenden Gasgemisch zu erhöhen.

Diese Aufgabe wird durch die unabhängigen Patentansprüche gelöst. Vorteilhafte Ausgestaltungen sind in den abhängigen Patentansprüchen, der nachfolgenden Beschreibung und den Figuren wiedergegeben, wobei diese Ausgestaltungen jeweils für sich genommen oder Kombination von wenigstens zwei dieser Ausgestaltungen miteinander einen vorteilhaften und/oder weiterbildenden Aspekt der Erfindung darstellen können. Vorteilhafte Ausgestaltungen der Vorrichtung können dabei vorteilhaften Ausgestaltungen des Verfahrens entsprechen, und umgekehrt, selbst wenn hierauf im Einzelnen im Folgenden nicht explizit hingewiesen wird.

Eine erfindungsgemäße Vorrichtung zum Messen einer CH₄-Konzentration in einem Wasserdampf enthaltenden Gasgemisch weist wenigstens eine NDIR-Messeinrichtung und wenigstens eine bezüglich einer Strömung des Gasgemischs durch die Vorrichtung der NDIR-Messeinrichtung vorgeschaltet angeordnete und für Wasserdampf selektiv permeabel ausgebildete Gasführungseinheit zum Trocknen und/oder Anfeuchten des Gasgemischs auf, wobei die Gasführungseinheit derart ausgebildet ist, dass ein Wassergehalt des aus ihr austretende Gasgemischs einem Wassergehalt einer die Gasführungseinheit umgebenden, CH₄-freien Umgebungsluft entspricht oder um maximal 5% von dem Wassergehalt der die Gasführungseinheit umgebenden, CH₄-freien Umgebungsluft abweicht.

Mit der erfindungsgemäßen Vorrichtung ist es möglich, ein der NDIR-Messeinrichtung zuzuführendes, CH₄ (Methan) enthaltendes Messgas mittels der Gasführungseinheit zu trocknen und/oder anzufeuchten, und zwar derart, dass der Wassergehalt des aus der Gasführungseinheit austretenden Gasgemischs dem Wassergehalt der die Gasführungseinheit umgebenden, CH₄-freien Umgebungsluft entspricht oder um maximal 5% von dem Wassergehalt der die Gasführungseinheit umgebenden, CH₄-freien Umgebungsluft abweicht. Letzteres kann dadurch gegeben sein, dass der Wassergehalt des der NDIR-Messeinrichtung zugeführten Messgases geringfügig höher oder tiefer ist als der Wassergehalt des der NDIR-Messeinrichtung zugeführten Nullgases. Die Erfindung macht es damit insbesondere möglich, eine Justiermessung einfach unter Verwendung der CH₄-freien Umgebungsluft durchzuführen, indem die Umgebungsluft (Nullgas) statt des Messgases für einen vorgegebenen Zeitraum durch die Gasführungseinheit und die NDIR-Messeinrichtung geführt wird und am Ende des Zeitraums wenigstens ein Messkanal der NDIR-Messeinrichtung zum Messen der CH₄-Konzentration auf den Wert Null gesetzt wird. Dabei weisen das der NDIR-Messeinrichtung zugeführte Messgas und das der NDIR-Messeinrichtung zugeführte Nullgas denselben bzw. um maximal 5% voneinander abweichenden Wassergehalt auf.

Wenn das der NDIR-Messeinrichtung zugeführte Messgas und das der NDIR-Messeinrichtung zugeführte Nullgas denselben Wassergehalt aufweisen, entspricht eine durch den Wasserdampf in dem Messgas verursachte Störung bei der Messung der CH₄-Konzentration in dem Wasserdampf enthaltenden Gasgemisch der durch den Wasserdampf in dem Nullgas verursachten Störung bei der Justiermessung. Wird von dem Messergebnis der NDIR-Messung das Messergebnis der Justiermessung subtrahiert, wird der störende Einfluss des Wasserdampfs in dem Messgas vollständig kompensiert, so dass die NDIR-Messung der CH₄-Konzentration in dem Wasserdampf enthaltenden Gasgemisch eine höhere Genauigkeit aufweist.

Wenn das der NDIR-Messeinrichtung zugeführte Messgas und das der NDIR-Messeinrichtung zugeführte Nullgas einen um maximal 5% voneinander abweichenden Wassergehalt aufweisen, entspricht eine durch den Wasserdampf in dem Messgas verursachte Störung bei der Messung der CH₄-Konzentration in dem Wasserdampf enthaltenden Gasgemisch im Wesentlichen der durch den Wasserdampf in dem Nullgas verursachten Störung bei der Justiermessung. Wird von dem Messergebnis der NDIR-Messung das Messergebnis der Justiermessung subtrahiert, wird der störende Einfluss des Wasserdampfs in dem Messgas weitestgehend kompensiert, so dass die NDIR-Messung der CH₄-Konzentration in dem Wasserdampf enthaltenden Gasgemisch eine höhere Genauigkeit aufweist. Die Gasführungseinheit kann derart ausgebildet sein, dass ein Betrag einer Differenz zwischen dem Wassergehalt des der NDIR-Messeinrichtung zugeführten Messgases und dem Wassergehalt des der NDIR-Messeinrichtung zugeführten Nullgas derart gering ist, dass die hierdurch bewirkte Abweichung der NDIR-Messung der CH₄-Konzentration in dem Wasserdampf enthaltenden Gasgemisch kleiner als 1% eines diesbezüglichen Messbereichsendwerts der NDIR-Messeinrichtung ist.

Der Zeitraum, in dem zur Justierung der NDIR-Messeinrichtung das Nullgas der NDIR-Messeinrichtung zugeführt wird, kann beispielsweise im Sekundenbereich liegen, beispielsweise in einem Bereich von 2 Sekunden bis 60 Sekunden. Dieser Justierzeitraum kann beispielsweise über eine Geräteelektronik vorgegeben und über eine mit der Geräteelektronik ausführbare Software eingestellt werden.

Für die NDIR-Messung der CH₄-Konzentration in dem Wasserdampf enthaltenden Gasgemisch kann das Messgas für einen vorgegebenen Messzeitraum durch die NDIR-Messeinrichtung geführt werden, der in einem Stundenbereich, einem Minutenbereich oder einem Sekundenbereich liegen kann. Der Messzeitraum kann beispielsweise derart kurz gewählt werden, dass in dem Messzeitraum eine CH₄-Konzentrationsdrift Δc kleiner als 1% vom Messbereichsendwert der NDIR-Messeinrichtung ist.

Am Ende des Justierzeitraums kann jeder Messkanal der NDIR-Messeinrichtung, insbesondere der Messkanal zum Messen der CH₄-Konzentration in dem Wasserdampf enthaltenden Gasgemisch, der in einem vorhergehenden Messzeitraum um einen Konzentrationsbetrag Δc gedriftet sein kann, auf den Wert Null gesetzt werden (Δc=0). Hierdurch kann zusätzlich auch die Nullpunktdrift kompensiert werden.

Die erfindungsgemäße Vorrichtung kann beispielsweise derart betrieben werden, dass nach einer Messphase, also einer NDIR-Messung der CH₄-Konzentration in dem Wasserdampf enthaltenden Gasgemisch, für einen vorgegebenen Zeitraum das Nullgas durch die Gasführungseinheit und die NDIR-Messeinrichtung geführt wird. Anschließend kann ein weiterer Messvorgang durchgeführt werden, so dass Messvorgänge und Justiervorgänge beispielsweise abwechselnd durchgeführt werden können, wodurch sich die Vorrichtung beispielsweise für eine dauerhafte Überwachung der CH₄-Konzentration einer Abluft, der das den Wasserdampf enthaltende Gasgemisch für jeden Messvorgang entnommen werden kann, eignet.

Eine Änderung des Wassergehalts der Umgebungsluft wirkt sich dabei nicht nachteilig auf die Genauigkeit der mit der Vorrichtung durchführbaren CH₄-Konzentrationsmessung aus, da mittels der Gasführungseinheit immer dafür gesorgt wird, dass das aus der Gasführungseinheit austretende Messgas denselben Wassergehalt oder annähernd denselben Wassergehalt wie die Umgebungsluft bzw. das Nullgas aufweist.

Die NDIR-Messeinrichtung kann wenigstens eine Infrarot-Strahlungsquelle, wenigstens eine Messküvette und wenigstens einen infrarotempfindlichen Strahlungssensor aufweisen. Die von der Infrarot-Strahlungsquelle emittierte Strahlung durchläuft die Messküvette, in der sich ein zu untersuchendes Gasgemisch befindet, und trifft am entgegengesetzten Ende der Messküvette auf den infrarotempfindlichen Strahlungssensor. Die Messküvette kann bezogen auf die Strahlrichtung der durch sie geführten IR-Strahlung eingangsseitig und ausgangsseitig jeweils ein für die IR-Strahlung durchlässiges Fenster aufweisen, das beispielsweise aus Calciumfluorid (CaF₂) hergestellt sein kann. Es zeigt sich, dass sich ein in der Messküvette befindlicher Wasserdampf in bzw. an dem Fenstermaterial anlagern kann, was zu einer Änderung der Transmissionseigenschaften des Fensters führt. Diese Änderung geht mit einer Änderung der spektralen Verteilung der aus der Messküvette austretenden elektromagnetischen Strahlung einher. Zudem wird die durch das jeweilige Küvettenfenster verlaufende elektromagnetische Strahlung durch die Einlagerung bzw. Anlagerung des Wasserdampfs in bzw. an dem Fenstermaterial zeitlich verzögert (sogenannter Hang up Effekt), wobei diese zeitliche Verzögerung durch Adsorption- und Desorptionseffekte an dem Küvettenfenster hervorgerufen wird. Auch diese störenden Effekte können mit der erfindungsgemäßen Vorrichtung zuverlässig und weitestgehend bis vollständig kompensiert werden. Insofern kann die erfindungsgemäße Vorrichtung zur Kompensation von Störungen bei einer Messung einer CH₄-Konzentration in einem Wasserdampf enthaltenden Gasgemisch verwendet werden, die durch eine Einlagerung und/oder Anlagerung von Wasserdampf in bzw. an einem Fenstermaterial eines Fensters einer Messküvette verursacht werden.

Die NDIR-Messeinrichtung kann auch wenigstens einen weiteren Messkanal zum Messen der Konzentration von wenigstens einer weiteren Gaskomponente des Wasserdampf enthaltenden Gasgemischs aufweisen. Die weitere Gaskomponente kann beispielsweise Distickstoffmonoxid (N₂O) oder Kohlendioxid (CO₂) sein. Die NDIR-Messeinrichtung kann sowohl einen Messkanal für Distickstoffmonoxid als auch einen Messkanal für Kohlendioxid aufweisen.

Die Gasführungseinheit zum Trocknen und/oder Anfeuchten des Gasgemischs kann beispielsweise rohrartig oder schlauchartig ausgebildet sein. Die Gasführungseinheit kann beispielsweise auch einem perfluorierten Copolymer mit ionischen Eigenschaften hergestellt sein, wobei als ionische Gruppe eine Sulfogruppe fungieren kann. Beispielsweise kann die Gasführungseinheit teilweise oder vollständig aus NAFION^{®} hergestellt sein. Die Gasführungseinheit kann ein einziges Gasführungsrohr bzw. einen einzigen Gasführungsschlauch oder zwei oder mehr parallel geschaltete Gasführungsrohre bzw. Gasführungsschläuche aufweisen. Befindet sich im Inneren der Gasführungseinheit eine hohe relative Feuchte und außerhalb der Gasführungseinheit eine geringe relative Feuchte, so tritt der Wasserdampf von innen nach außen durch die Wandung der Gasführungseinheit hindurch (Trocknung). Befindet sich hingegen im Inneren der Gasführungseinheit eine geringe relative Feuchte und außerhalb der Gasführungseinheit eine höhere relative Feuchte, so tritt der Wasserdampf von außen nach innen durch die Wandung der Gasführungseinheit hindurch (Anfeuchtung). Dieser Durchlass kann erfolgen bis der Wasserdampfpartialdruck im Inneren bzw. außerhalb der Gasführungseinheit und außerhalb bzw. im Inneren der Gasführungseinheit identisch ist. Es findet also ein Ausgleich der relativen Feuchte (RH) statt. Eine Länge der Gasführungseinheit ist vorzugsweise derart gewählt, dass der Feuchteaustausch zwischen Innen und Außen ≥95% ist. Dies ist so zu verstehen, dass die anfängliche Differenz zwischen der Feuchte des Messgases bzw. eines nachfolgend beschriebenen Prüfgases einerseits und der Umgebungsluft andererseits vor dem Eintreten des Messgases bzw. Prüfgases in die Gasführungseinheit 100% beträgt und am Ausgang der Gasführungseinheit lediglich noch ≤5% ist, wodurch der Feuchteaustausch zwischen Innen und Außen ≥95% beträgt. Dabei ist die Länge der Gasführungseinheit vorzugsweise in Abhängigkeit des Gasdurchflusses *V̇* durch die Gasführungseinheit gewählt, und zwar derart, dass die Länge der Gasführungseinheit mit steigendem Gasdurchfluss umso größer gewählt wird. Beispielsweise kann bei einem Gasdurchfluss von <1 Liter/Minute die Länge der Gasführungseinheit bei etwa 50 cm liegen.

Die Gasführungseinheit kann zusätzlich beispielsweise derart ausgebildet sein, dass ein Wassergehalt eines aus ihr austretenden, zu einer Endpunktjustage verwendeten Prüfgases einem Wassergehalt der die Gasführungseinheit umgebenden, CH₄-freien Umgebungsluft entspricht oder um maximal 5% von dem Wassergehalt der die Gasführungseinheit umgebenden, CH₄-freien Umgebungsluft abweicht. Eine Endpunktjustage wird in der Regel nur alle paar Monate oder einmal im Jahr durchgeführt. Da das für die Endpunktjustage verwendete Prüfgas üblicherweise sehr trocken ist, sollte die Feuchte bzw. der Wassergehalt des Prüfgases ebenfalls an die Feuchte der Umgebungsluft angeglichen werden. Der Wassergehalt des durch die Gasführungseinheit strömenden Prüfgases würde somit an den Wassergehalt der Umgebungsluft angepasst, indem Feuchte aus der Umgebungsluft durch die Gasführungseinheit hindurch auf das Prüfgas übertragen wird (Anfeuchtung). Das Prüfgas würde dann auf dieselbe Art und Weise geführt wie das Messgas. Die Endpunktjustage kann manuell oder automatisch durchgeführt werden.

Die erfindungsgemäße Vorrichtung zum Messen einer CH₄-Konzentration in einem Wasserdampf enthaltenden Gasgemisch kann beispielsweise für eine dauerhafte Methanmessung in Kläranlagen und Kuhställen verwendet werden. Dabei kann beispielsweise als CH₄-freie Umgebungsluft Luft aus der Umgebung einer Kläranlage bzw. eines Kuhstalls verwendet werden.

Gemäß einer vorteilhaften Ausgestaltung weist die Vorrichtung wenigstens eine der Gasführungseinheit vorgeschaltet angeordnete, elektrisch ansteuerbare Ventileinheit auf, über die der Gasführungseinheit je nach Schaltzustand der Ventileinheit entweder für eine Justierung der NDIR-Messeinrichtung die Umgebungsluft oder für eine CH₄-Konzentrationsmessung das Gasgemisch zuführbar ist. Die Ventileinheit kann mittels einer Geräteelektronik der Vorrichtung angesteuert werden, beispielsweise um Messvorgänge und Justiervorgänge automatisiert und im Wechsel durchführen zu können. Hierbei kann die Ventileinheit wenigstens einen Zugang für CH₄-freie Umgebungsluft, wenigstens einen Zugang für das Messgas und wenigstens einen Abgang aufweisen, über den entweder das Messgas oder die Umgebungsluft von der Ventileinheit abgeht, um zu der Gasführungseinheit strömen zu können.

Gemäß einer weiteren vorteilhaften Ausgestaltung weist die Ventileinheit wenigstens ein 2/3-Wegeventil auf. Hierdurch lässt sich die Vorrichtung baulich kompakt realisieren. Stattdessen kann die Ventileinheit wenigstens ein Ventil für das Messgas und wenigstens ein Ventil für die CH₄-freie Umgebungsluft aufweisen.

Gemäß einer weiteren vorteilhaften Ausgestaltung weist die Vorrichtung wenigstens eine der Ventileinheit, der Gasführungseinheit oder der NDIR-Messeinrichtung nachgeschaltet angeordnete Fördereinheit auf, mittels der je nach Schaltzustand der Ventileinheit entweder die Umgebungsluft oder das Gasgemisch durch die NDIR-Messeinrichtung förderbar ist. Hierdurch kann die Vorrichtung mit lediglich einer entsprechend angeordneten Fördereinheit ausgestattet sein, um die jeweiligen Luft- bzw. Gasströmungen durch die Vorrichtung zu bewirken. Dadurch lässt sich die Vorrichtung baulich kompakt realisieren. Zudem können die Volumenströme der Luft- bzw. Gasströmungen durch die Vorrichtung mittels einer einzigen Fördereinheit auf einfache Art und Weise im Wesentlichen gleich gehalten werden. Alternativ können wenigstens eine Fördereinheit für das Messgas und wenigstens eine weitere Fördereinheit für die CH₄-freie Umgebungsluft vorhanden sein.

Gemäß einer weiteren vorteilhaften Ausgestaltung weist die Vorrichtung wenigstens eine der Ventileinheit vorgeschaltete Filtereinheit zum Filtern der Umgebungsluft auf, mit der wenigstens eine eine CH₄-Konzentrationsmessung störende Gaskomponente der Umgebungsluft aus der Umgebungsluft herausfilterbar ist. Beispielsweise können in der CH₄-freien Umgebungsluft Gasspuren enthalten sein, die den oben beschriebenen Nullabgleich (Justierung) von wenigstens einem Messkanal der NDIR-Messeinrichtung stören könnten. Diese Gasspuren können mit der Filtereinheit herausgefiltert werden. Die Filtereinheit kann als chemischer Absorber (sogenannter Scrubber) ausgebildet sein.

Gemäß einer weiteren vorteilhaften Ausgestaltung weist die Vorrichtung wenigstens eine Geräteelektronik auf, die eingerichtet ist, die Ventileinheit derart anzusteuern, dass zunächst für die Justierung der NDIR-Messeinrichtung die Umgebungsluft der Gasführungseinheit zuführbar ist und nach Abschluss der Justierung der NDIR-Messeinrichtung für die CH₄-Konzentrationsmessung das Gasgemisch der Gasführungseinheit zuführbar ist. Über die Geräteelektronik kann die vorbeschriebene Funktionsweise der Vorrichtung automatisiert werden. Hierzu kann die Geräteelektronik wenigstens einen Prozessor aufweisen, auf dem eine geeignete Software ausführbar ist. Die Geräteelektronik kann eingerichtet sein, die Ventileinheit derart anzusteuern, dass die Justierung und die CH₄-Konzentrationsmessung dauerhaft im Wechsel durchgeführt werden können.

Gemäß einer weiteren vorteilhaften Ausgestaltung ist die Geräteelektronik eingerichtet, am Ende der Justierung der NDIR-Messeinrichtung alle Messkanäle der NDIR-Messeinrichtung auf den Wert Null zu setzen. Mit dieser Ausgestaltung sind die oben mit Bezug auf die entsprechende Ausgestaltung der Vorrichtung genannten Vorteile entsprechend verbunden.

Mit der Erfindung wird zudem eine Verwendung der Vorrichtung nach einer der vorgenannten Ausgestaltungen oder einer Kombination von wenigstens zwei dieser Ausgestaltungen miteinander zum Messen einer CH₄-Konzentration in einem Wasserdampf enthaltenden Gasgemisch vorgeschlagen.

Mit dieser Verwendung sind die oben mit Bezug auf die Vorrichtung genannten Vorteile entsprechend verbunden.

Gemäß einem erfindungsgemäßen Verfahren zum Messen einer CH₄-Konzentration in einem Wasserdampf enthaltenden Gasgemisch mittels wenigstens einer NDIR-Messeinrichtung wird das Gasgemisch mittels wenigstens einer bezüglich einer Strömung des Gasgemischs der NDIR-Messeinrichtung vorgeschaltet angeordneten und für Wasserdampf selektiv permeablen Gasführungseinheit getrocknet oder angefeuchtet, derart, dass ein Wassergehalt des aus der Gasführungseinheit austretenden Gasgemischs einem Wassergehalt einer die Gasführungseinheit umgebenden, CH₄-freien Umgebungsluft entspricht.

Mit dem Verfahren sind die oben mit Bezug auf die Vorrichtung genannten Vorteile entsprechend verbunden. Insbesondere kann die Vorrichtung gemäß einer der oben genannten Ausgestaltungen oder einer Kombination von wenigstens zwei dieser Ausgestaltungen miteinander zur Durchführung des Verfahrens verwendet werden.

Gemäß einer vorteilhaften Ausgestaltung wird der Gasführungseinheit entweder für eine Justierung der NDIR-Messeinrichtung die Umgebungsluft oder für eine CH₄-Konzentrationsmessung das Gasgemisch zugeführt. Mit dieser Ausgestaltung sind die oben mit Bezug auf die entsprechende Ausgestaltung der Vorrichtung genannten Vorteile entsprechend verbunden.

Gemäß einer weiteren vorteilhaften Ausgestaltung wird wenigstens eine eine CH₄-Konzentrationsmessung störende Gaskomponente der Umgebungsluft aus der Umgebungsluft herausgefiltert. Mit dieser Ausgestaltung sind die oben mit Bezug auf die entsprechende Ausgestaltung der Vorrichtung genannten Vorteile entsprechend verbunden.

Gemäß einer weiteren vorteilhaften Ausgestaltung wird zunächst für die Justierung der NDIR-Messeinrichtung die Umgebungsluft der Gasführungseinheit zugeführt und nach Abschluss der Justierung der NDIR-Messeinrichtung für die CH₄-Konzentrationsmessung das Gasgemisch der Gasführungseinheit zugeführt. Mit dieser Ausgestaltung sind die oben mit Bezug auf die entsprechende Ausgestaltung der Vorrichtung genannten Vorteile entsprechend verbunden.

Gemäß einer weiteren vorteilhaften Ausgestaltung werden am Ende der Justierung der NDIR-Messeinrichtung alle der CH₄-Konzentrationsmessung dienenden Messkanäle der NDIR-Messeinrichtung auf den Wert Null gesetzt. Mit dieser Ausgestaltung sind die oben mit Bezug auf die entsprechende Ausgestaltung der Vorrichtung genannten Vorteile entsprechend verbunden.

Im Folgenden wird die Erfindung unter Bezugnahme auf die beigefügten Figuren anhand einer bevorzugten Ausführungsform beispielhaft erläutert, wobei die nachfolgend erläuterten Merkmale sowohl jeweils für sich genommen als auch in unterschiedlicher Kombination miteinander einen vorteilhaften und/oder weiterbildenden Aspekt der Erfindung darstellen können.

### Kurze Beschreibung der Figuren

### Es zeigt:

- Fig. 1: eine schematische Darstellung eines Ausführungsbeispiels für eine erfindungsgemäße Vorrichtung; und
- Fig. 2: ein Diagramm zur Funktion der Gasführungseinheit der in Fig. 1 gezeigten Vorrichtung.

### Ausführliche Beschreibung der Figuren

In den Figuren sind gleiche bzw. funktionsgleiche Bauteile mit denselben Bezugszeichen versehen. Eine wiederholte Beschreibung solcher Bauteile kann nachfolgend zur Vermeidung von unnötigen Wiederholungen im Einzelnen weggelassen sein.

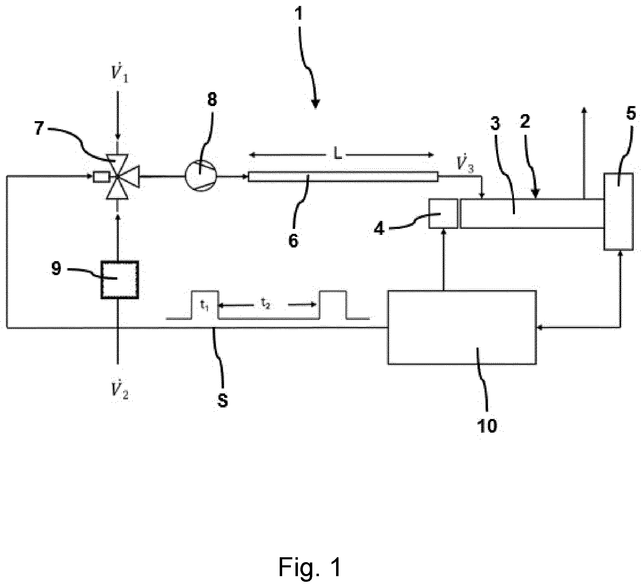

Fig. 1 zeigt eine schematische Darstellung eines Ausführungsbeispiels für eine erfindungsgemäße Vorrichtung 1 zum Messen einer CH₄-Konzentration in einem Wasserdampf enthaltenden Gasgemisch.

Die Vorrichtung 1 weist eine NDIR-Messeinrichtung 2 auf. Die NDIR-Messeinrichtung 2 weist eine Messküvette 3, eine IR-Strahlungsquelle 4 und einen IR-Strahlungssensor 5 auf. Die Messküvette 3 weist einen nicht detaillierter gezeigten Zugang auf, über den der Messküvette 3 ein Gas mit einem Volumenstrom *V̇*₃ zuführbar ist. Des Weiteren weist die Messküvette 3 einen nicht detaillierter gezeigten Ausgang auf, über den ein Gas aus der Messküvette 3 entsprechend dem Pfeil P abführbar ist. Die Messküvette 3 weist bezüglich einer von der IR-Strahlungsquelle 4 zu dem IR-Strahlungssensor 5 verlaufenden, nicht gezeigten elektromagnetischen Strahlung eingangsseitig und ausgangsseitig jeweils ein nicht gezeigtes, für IR-Strahlung durchlässiges Fenster auf, das aus Calciumfluorid hergestellt ist.

Die Vorrichtung 1 weist zudem eine bezüglich einer Strömung des Gasgemischs durch die Vorrichtung 1 der NDIR-Messeinrichtung 2 vorgeschaltet angeordnete und für Wasserdampf selektiv permeabel ausgebildete Gasführungseinheit 6 zum Trocknen und/oder Anfeuchten des Gasgemischs auf. Die Gasführungseinheit 6 ist als NAFION^{®}-Schlauch mit einer Länge L ausgebildet.

Die Gasführungseinheit 6 ist derart ausgebildet, dass ein Wassergehalt des aus ihr austretenden Gasgemischs entweder einem Wassergehalt einer die Gasführungseinheit 6 umgebenden, CH₄-freien Umgebungsluft entspricht oder um maximal 5% von dem Wassergehalt der die Gasführungseinheit 6 umgebenden, CH₄-freien Umgebungsluft abweicht.

Zudem weist die Vorrichtung 1 eine der Gasführungseinheit 6 vorgeschaltet angeordnete, elektrisch ansteuerbare Ventileinheit 7 auf, über die der Gasführungseinheit 6 je nach Schaltzustand der Ventileinheit 7 entweder für eine Justierung der NDIR-Messeinrichtung 2 die Umgebungsluft mit einem Volumenstrom *V̇*₂ oder für eine CH₄-Konzentrationsmessung das Gasgemisch mit einem Volumenstrom *V̇*₁ zuführbar ist. Hierzu ist die Ventileinheit 7 als 2/3-Wegeventil und als Magnetventil ausgebildet.

Darüber hinaus weist die Vorrichtung 1 eine der Ventileinheit 7 nachgeschaltet angeordnete und der Gasführungseinheit 6 vorgeschaltet angeordnete Fördereinheit 8 auf, mittels der je nach Schaltzustand der Ventileinheit 7 entweder die Umgebungsluft oder das Gasgemisch durch die NDIR-Messeinrichtung 2 förderbar ist. Die Fördereinheit 8 ist als Pumpe ausgebildet. Durch die spezielle Anordnung der Fördereinheit 8 gilt: *V̇*₁ = *V̇*₂ *= V̇*₃.

Des Weiteren weist die Vorrichtung 1 eine der Ventileinheit 7 bezüglich einer Umgebungsluftströmung vorgeschaltete Filtereinheit 9 zum Filtern der Umgebungsluft auf, mit der wenigstens eine eine CH₄-Konzentrationsmessung störende Gaskomponente der Umgebungsluft aus der Umgebungsluft herausfilterbar ist. Hierzu ist die Filtereinheit 9 als chemischer Absorber ausgebildet.

Ferner weist die Vorrichtung 1 eine Geräteelektronik 10 auf, die eingerichtet ist, die Ventileinheit 7 bei eingeschalter Fördereinheit 8 derart anzusteuern, dass zunächst für die Justierung der NDIR-Messeinrichtung 2 die Umgebungsluft der Gasführungseinheit 6 zuführbar ist und nach Abschluss der Justierung der NDIR-Messeinrichtung 2 für die CH₄-Konzentrationsmessung das Gasgemisch der Gasführungseinheit 6 zuführbar ist. Hierzu ist die Geräteelektronik 10 über eine Steuerleitungen S mit der Ventileinheit 7 verbunden.

Dabei ist die Geräteelektronik 10 eingerichtet, für einen Zeitraum t₁ der Gasführungseinheit 6 für die Justierung der NDIR-Messeinrichtung 2 die Umgebungsluft zuzuführen und für einen Zeitraum t₂ der Gasführungseinheit 6 nach Abschluss der Justierung der NDIR-Messeinrichtung 2 für die CH₄-Konzentrationsmessung das Gasgemisch der Gasführungseinheit 6 zuzuführen, wobei der Zeitraum t₂ länger als der Zeitraum t₂ ist. Die Geräteelektronik 10 ist eingerichtet, diese Zeiträume im Wechsel zu realisieren.

Zudem ist die Geräteelektronik 10 eingerichtet, am Ende der Justierung der NDIR-Messeinrichtung 2 alle Messkanäle der NDIR-Messeinrichtung 2 auf den Wert Null zu setzen. Die Geräteelektronik 10 kann auch als Teil der NDIR-Messeinrichtung 2 dienen.

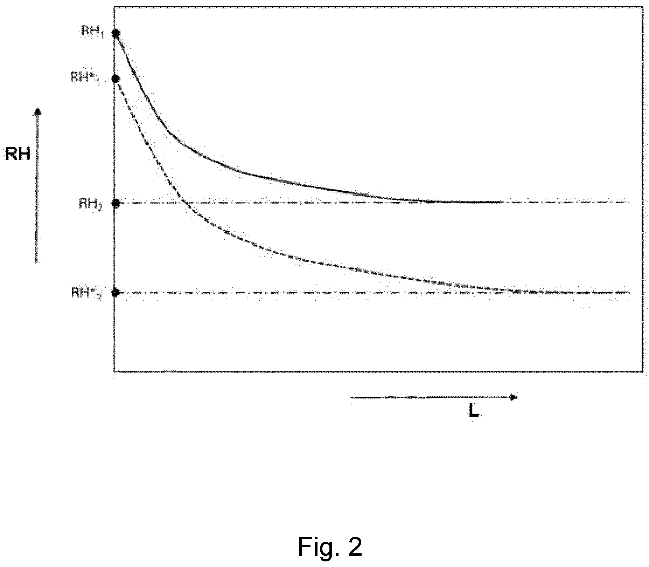

Fig. 2 zeigt ein Diagramm zur Funktion der Gasführungseinheit 6 der in Fig. 1 gezeigten Vorrichtung 1. Es ist die relative Feuchte RH gegen die Länge L der Gasführungseinheit 6 aufgetragen. Fig. 2 wird in einer Zusammenschau mit Fig. 1 erläutert.

Beträgt die relative Feuchte der CH₄-freien Umgebungsluft RH₂ und wird ein Messgas mit der relativen Feuchte RH₁ durch die Gasführungseinheit 6 geführt, passt sich die relative Feuchte RH₁ des Messgases an die relative Feuchte RH₁ der Umluft an, was bedeutet, dass das Messgas mittels der Gasführungseinheit 6 getrocknet wird. Je größer die Länge L der Gasführungseinheit 6 ist, desto mehr gleich sich die relative Feuchte RH₁ des Messgases an die relative Feuchte RH₁ der Umgebungsluft an.

In Fig. 2 ist eine weitere Situation gezeigt, in der sich die relative Feuchte der CH₄-freien Umgebungsluft auf den Wert RH*₂ verringert hat. Dann nähert sich die relative Feuchte RH*₁ des Messgases an die relative Feuchte RH*₂ der Umgebungsluft an. Die Wirkung der Vorrichtung 1 ist also unabhängig von der momentanen relativen Feuchte RH*₂ der Umgebungsluft.

### Bezugszeichenliste

- 1: Vorrichtung
- 2: NDIR-Messeinrichtung
- 3: Messküvette
- 4: IR-Strahlungsquelle
- 5: IR-Strahlungssensor
- 6: Gasführungseinheit
- 7: Ventileinheit
- 8: Fördereinheit
- 9: Filtereinheit
- 10: Geräteelektronik
- L: Länge von 6
- P: Pfeil (Auslass)
- RH: relative feuchte
- RH₁: relative Feuchte (Messgas)
- RH*₁: relative Feuchte (Messgas)
- RH₂: relative Feuchte (Umgebungsluft)
- RH*₂: relative Feuchte (Umgebungsluft)
- S: Steuerleitung
- t₁: Zeitraum (Justierung)
- t₂: Zeitraum (Messung)
- *V̇*₁: Volumenstrom (Messgas)
- *V̇*₂: Volumenstrom (Umgebungsluft)
- *V̇*₃: Volumenstrom

## Patentansprüche

1. Vorrichtung (1) zum Messen einer CH₄-Konzentration in einem Wasserdampf enthaltenden Gasgemisch, aufweisend wenigstens eine NDIR-Messeinrichtung (2) und wenigstens eine bezüglich einer Strömung des Gasgemischs durch die Vorrichtung (1) der NDIR-Messeinrichtung (2) vorgeschaltet angeordnete und für Wasserdampf selektiv permeabel ausgebildete Gasführungseinheit (6) zum Trocknen und/oder Anfeuchten des Gasgemischs, wobei die Gasführungseinheit (6) derart ausgebildet ist, dass ein Wassergehalt des aus ihr austretenden Gasgemischs entweder einem Wassergehalt einer die Gasführungseinheit (6) umgebenden, CH₄-freien Umgebungsluft entspricht oder um maximal 5% von dem Wassergehalt der die Gasführungseinheit (6) umgebenden, CH₄-freien Umgebungsluft abweicht.

2. Vorrichtung (1) nach Anspruch 1, **gekennzeichnet durch** wenigstens eine der Gasführungseinheit (6) vorgeschaltet angeordnete, elektrisch ansteuerbare Ventileinheit (7), über die der Gasführungseinheit (6) je nach Schaltzustand der Ventileinheit (7) entweder für eine Justierung der NDIR-Messeinrichtung (2) die Umgebungsluft oder für eine CH₄-Konzentrationsmessung das Gasgemisch zuführbar ist.

3. Vorrichtung (1) nach Anspruch 2, **dadurch gekennzeichnet, dass** die Ventileinheit (7) wenigstens ein 2/3-Wegeventil aufweist.

4. Vorrichtung (1) nach Anspruch 2 oder 3, **gekennzeichnet durch** wenigstens eine der Ventileinheit (7), der Gasführungseinheit (6) oder der NDIR-Messeinrichtung (2) nachgeschaltet angeordnete Fördereinheit (8), mittels der je nach Schaltzustand der Ventileinheit (7) entweder die Umgebungsluft oder das Gasgemisch durch die NDIR-Messeinrichtung (2) förderbar ist.

5. Vorrichtung (1) nach einem der Ansprüche 2 bis 4, **gekennzeichnet durch** wenigstens eine der Ventileinheit (7) vorgeschaltete Filtereinheit (9) zum Filtern der Umgebungsluft, mit der wenigstens eine eine CH₄-Konzentrationsmessung störende Gaskomponente der Umgebungsluft aus der Umgebungsluft herausfilterbar ist.

6. Vorrichtung (1) nach einem der Ansprüche 2 bis 5, **gekennzeichnet durch** wenigstens eine Geräteelektronik (10), die eingerichtet ist, die Ventileinheit (7) derart anzusteuern, dass zunächst für die Justierung der NDIR-Messeinrichtung (2) die Umgebungsluft der Gasführungseinheit (6) zuführbar ist und nach Abschluss der Justierung der NDIR-Messeinrichtung (2) für die CH₄-Konzentrationsmessung das Gasgemisch der Gasführungseinheit (6) zuführbar ist.

7. Vorrichtung (1) nach Anspruch 6, **dadurch gekennzeichnet, dass** die Geräteelektronik (10) eingerichtet ist, am Ende der Justierung der NDIR-Messeinrichtung (2) alle Messkanäle der NDIR-Messeinrichtung (2) auf den Wert Null zu setzen.

8. Verwendung der Vorrichtung (1) nach einem der Ansprüche 1 bis 7 zum Messen einer CH₄-Konzentration in einem Wasserdampf enthaltenden Gasgemisch.

9. Verfahren zum Messen einer CH₄-Konzentration in einem Wasserdampf enthaltenden Gasgemisch mittels wenigstens einer NDIR-Messeinrichtung (2), wobei das Gasgemisch mittels wenigstens einer bezüglich einer Strömung des Gasgemischs der NDIR-Messeinrichtung (2) vorgeschaltet angeordneten und für Wasserdampf selektiv permeablen Gasführungseinheit (6) getrocknet oder angefeuchtet wird, derart, dass ein Wassergehalt des aus der Gasführungseinheit (6) austretenden Gasgemischs einem Wassergehalt einer die Gasführungseinheit (6) umgebenden, CH₄-freien Umgebungsluft entspricht.

10. Verfahren nach Anspruch 9, **dadurch gekennzeichnet, dass** der Gasführungseinheit (2) entweder für eine Justierung der NDIR-Messeinrichtung (2) die Umgebungsluft oder für eine CH₄-Konzentrationsmessung das Gasgemisch zugeführt wird.

11. Verfahren nach Anspruch 10, **dadurch gekennzeichnet, dass** wenigstens eine eine CH₄-Konzentrationsmessung störende Gaskomponente der Umgebungsluft aus der Umgebungsluft herausgefiltert wird.

12. Verfahren nach einem der Ansprüche 9 bis 11, **dadurch gekennzeichnet, dass** zunächst für die Justierung der NDIR-Messeinrichtung (2) die Umgebungsluft der Gasführungseinheit (6) zugeführt wird und nach Abschluss der Justierung der NDIR-Messeinrichtung (2) für die CH₄-Konzentrationsmessung das Gasgemisch der Gasführungseinheit (6) zugeführt wird.

13. Verfahren nach einem der Ansprüche 10 bis 12, **dadurch gekennzeichnet, dass** am Ende der Justierung der NDIR-Messeinrichtung (2) alle der CH₄-Konzentrationsmessung dienenden Messkanäle der NDIR-Messeinrichtung (2) auf den Wert Null gesetzt werden.
